(19)

Europäisches Patentamt
European Patent Office
Office européen des brevets

(11) **EP 3 679 943 A1**

(12) **EUROPEAN PATENT APPLICATION**
published in accordance with Art. 153(4) EPC

(43) Date of publication:
**15.07.2020 Bulletin 2020/29**

(21) Application number: **18853969.6**

(22) Date of filing: **23.07.2018**

(51) Int Cl.:
*A61K 38/21* [(2006.01)]    *A61K 47/60* [(2017.01)]
*A61P 31/12* [(2006.01)]    *B01J 19/08* [(2006.01)]

(86) International application number:
**PCT/RU2018/050082**

(87) International publication number:
**WO 2019/050437 (14.03.2019 Gazette 2019/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.09.2017 RU 2017131647**

(71) Applicant: **Obshchestvo S Ogranichennoy
Otvetstvennost
Yu "Scientific Fututre Management"
Novosibirskiy Rayon, R.P. Koltsovo 630059 (RU)**

(72) Inventors:
• **ARTAMONOV, Andrei Vladimirovich
g. Novosibirsk 630005 (RU)**

• **BEKAREV, Andrei Aleksandrovich
g. Novosibirsk 630090 (RU)**
• **DYGAI, Aleksandr Mikhailovich
g. Tomsk 634050 (RU)**
• **ZDANOV, Vadim Vadimovich
g. Tomsk 634061 (RU)**
• **KINSHT, Dmitrii Nikolaevich
630559 (RU)**
• **MADONOV, Pavel Gennad'evich
g. Novosibirsk 630048 (RU)**
• **SHERSTOBOEV, Evgenii Yur'evich
g. Tomsk 634034 (RU)**

(74) Representative: **Vitina, Maruta et al
Agency TRIA ROBIT
P.O. Box 22
1010 Riga (LV)**

(54) **PEGYLATED INTERFERON LAMBDA FOR ORAL ADMINISTRATION, AND METHOD FOR PRODUCING SAME**

(57)    The group of inventions is related to medicine, namely infectious diseases, as well as pharmacology, the pharmaceutical industry and biotechnology and provides pegylated interferon lambda having antiviral activity against hepatotropic viruses, wherein said agent has oral bioavailability at least 10%, which ensures high concentration of the agent in the liver, bypassing the systemic circulation, reduces systemic side effects and increases the compliance with therapy, and wherein the method for preparation of the agent comprises irradiating a solution of polyethylene glycol with a molecular weight of from about 400 to about 5,000 Da with ionizing radiation in a dose of 1 to 5 Mrad, mixing the resulting solution with an interferon lambda solution, freezing and cooling of the resulting mixture to a temperature of - 10 °C or below, and the irradiating of the frozen and cooled mixture with ionizing radiation in a dose of 0.1 to 0.5 Mrad.

FIG. 2

EP 3 679 943 A1

**Description**

**Field of the invention**

The group of inventions is related to medicine, namely infectious diseases, and pharmacology, as well as the pharmaceutical industry and biotechnology, and is intended to produce pegylated interferon lambda, which can be used to treat viral hepatites, since it has antiviral activity against hepatotropic viruses and high oral bioavailability, which ensures high concentration of the agentin the liver, bypassing the systemic circulation, reduces systemic side effects and increases compliance with therapy.

**State of art**

Interferons lambda (IFN-$\lambda$) form a family of interferons of the third type, which consists of four members:IFN-$\lambda$1, IFN-$\lambda$2, IFN-$\lambda$3 and IFN-$\lambda$4, also denoted IL-29, IL-28A, IL-28B and IFN-$\lambda$4, respectively [1, 2, 3]. IFN-$\lambda$s belong to the family of cytokines and perform functions similar to the family of interferons of the first type (IFN-$\alpha$ and/or IFN-$\beta$), however, differing significantly from the latter: firstly, IFN-$\lambda$s act primarily in epithelial cells that are constantly exposed to synanthropic and pathogenic microorganisms, and secondly, IFN-$\lambda$s possess significant therapeutic benefits due to more targeted signal transduction, whereby a number of side effects limiting the clinical use of IFN-$\alpha$ and IFN-$\beta$ is eliminated. In addition, IFN-$\lambda$s have an immunomodulatory effects on the innate and adaptive branches of the immune system, which partially coincide with those of IFN-$\alpha$ and IFN-$\beta$ [4, 5, 6, 7].

Antiviral activity of IFN-$\lambda$ *in vivo* was firstly detected in 2006 against the herpes simplex virus of the second type [8]. Further studies in animal models revealed antiviral activity of exogenous IFN-$\lambda$ comparable to the effects of IFN-$\alpha$ and IFN-$\beta$ against such viruses as respiratory syncytial virus, rotavirus, foot and mouth disease virus, murine norovirus, West Nile fever virus, type 1 herpes simplex virus, influenza virus, human metapneumovirus, coronavirus, vaccinia virus, hepatitis B virus, murine pneumonia virus [1, 9, 10, 11, 12, 13, 14, 15, 16]. It was also found that the antiviral activity of IFN-$\lambda$ *in vivo* is more pronounced against viruses that infect the epithelial cells of the respiratory, gastrointestinal and urogenital tracts, and alsoof the liver [13]. This has led to interest in IFN-$\lambda$ as a potential therapeutic agent for viral hepatitis [17, 18].

Viral hepatites are a serious threat to public health on an international scale and imposes a significant burden on the population of all regions of the world. In 2016, the World Health Organization adopted the "Global Health Sector Strategy on Viral Hepatitis 2016-2021", which states that viral hepatitis takes a large number of human lives, and causes significant harm to local communities and health system. It accounts for approximately 1.4 million deaths per year due to acute infection, and also cancer and liver cirrhosis associated with hepatitis, which is comparable to death rates from HIV and tuberculosis. Of this number, approximately 47 % of deaths are caused by the hepatitis B virus, 48 % by the hepatitis C virus, and the rest by the hepatitis A and hepatitis E viruses. Viral hepatitis is increasingly becoming the cause of death for people living with HIV. Approximately 2.9 million people living with HIV are co-infected with the hepatitis C virus, and 2.6 million people are co-infected with the hepatitis B virus. Globally, approximately 240 million people are chronically infected with the hepatitis B virus and 130-150 million people with the hepatitis C virus. This strategy is aimed at combating all five hepatitis viruses (A, B, C, D, and E) and first of all, the hepatitis B and C viruses, taking into account their high public health importance. With regard to the treatment of chronic viral hepatitis B and C, the WHO headquarters support a public health approach that involves a transition to simpler and safer oral treatment regimens

Although the new guidelines in the updated "WHO Guidelines for the screening, care and treatment of persons with chronic hepatitis C infection" recommend use of direct-acting antiviral regimens, the combination of sofosbuvir / pegylated interferon and ribavirin is still recommended as an alternative treatment option for special populations[20]. Pegylated interferons provide a stable level of interferon in serum for seven days. Pegylated interferon-$\alpha$2b (PegIntron®, Schering-Plowcompany, USA) [21], pegylated interferon-$\alpha$2a (Pegasis®, Roche, Switzerland) [22], and cepegylated-interferon-$\alpha$2b (Algeron®, "Biocad", Russia) [23] are known. The main disadvantages of these drugs are:

-    weekly subcutaneous injections with therapy duration of 12 weeks or more, which significantly reduces compliance with therapy;
-    common side effects of treatment in the form of flu-like syndrome, arthralgia, depressed mood, diarrhea, depressed mood, hallucinations, skin rashes, allergy, diseases of the hematopoietic system, hair loss, which also reduces compliance with therapy[24, 25];
-    the formation of antibodies neutralizing the antiviral activity of the drug in 30 % of patients, which is accompanied by the development of resistance to recombinant interferons [26].

Therefore, there is an acute need for new therapeutic and prophylactic drugs against viral hepatites, including hepatitis C, which drugs would have high efficiency and minimal side effects.

One of the options for highly effective therapy of this disease is the use of drugs based on interferon lambda, since IFN-λs have more limited expression patterns and are expressed predominantly in hepatocytes. IFN-λs have an antiviral activity in the liver that is comparable to IFN-α, but with fewer side effects, which creates background for the therapeutic benefits of IFN-λ in the treatment of viral infections of the liver or liver conditions, including viral hepatitis C and viral hepatitis B [27].

The closest to the claimed agent in technical essence is the pegylated interferon lambda-based medication for the treatment and prevention of hepatitis C, which is described in the patent [28]. To date, it is the only pegylated interferon lambda-based medication in the world brought to clinical studies and known as BMS-914143 medication from "Bristol-Myers Squibb" company. The main disadvantage of this drug is the need in weekly parenteral administration, which, when recommended duration of the course of therapy is of 12 weeks or more, can cause reactions at the site of administration in the form of painfulness, redness, edema, infiltrates and even necrosis, which cannot meet the requirements of compliance with therapy. In addition, systemic administration is associated with common complications: fever, allergic and anaphylactic reactions.

Closest to the claimed method for preparation is the method described in the patent [29], which method comprises the dissolution of biologically active substance, for example interferon, in a solution of a water-soluble pharmacologically acceptable polymer in a concentration above 10 % to a concentration of a saturated solution and the irradiation of the reaction mixture with ionizing radiation. The disadvantage of this method is the uncontrolled formation of a covalent bond between the biologically active substance and the polymer under the effect of ionizing radiation, which can reduce the specific activity of interferon by blocking the active centers, as well as by irreversible changes in the structure of the protein molecule.

The technical result achieved by this group of the inventions is to expand the range of agents available for the treatment of infectious diseases caused by hepatotropic viruses, which agent could be used with other routes of the drug administration than parenteral and to increase compliance of patients with ongoingtherapy, as well as methods for preparation thereof.

## Summary of the Invention

The claimed technical result is achieved in an agent that represents pegylated interferon lambda (hereinafter PEG-IFN-λ) having antiviral activity against hepatotropic viruses, wherein oral bioavailability of said agent is at least 10 %, and said agent is obtained by irradiating a solution of polyethylene glycol with a molecular weight of from about 400 to about 5,000 Da with ionizing radiation in a dose of 1 to 5 Mrad, mixing the resulting solution with an interferon lambda solution, freezing and cooling of the obtained mixture to a temperature of -10 °C or below, and the irradiating frozen and cooled mixture with ionizing radiation in a dose of from 0.1 to 0.5 Mrad.

## Antiviral activity of PEG-IFN-λ

Antiviral properties of the proposed agent were revealed due to experimental studies carried out in the "cultural HCV (HCVcc) clone JFH1 / human hepatoma cells Huh7.5" system. At the date, this system is the most proper and has been adopted by FDA in the USA and by the European Community for a specific assessment of the anti-HCV activity of pharmaceuticals at the pre-clinical stage.

Evaluation of the antiviral activity of PEG-IFN-λ was performed using the culture of HCV strain isolated in 1999 in Japan from the serum of the patient with fulminant hepatitis. Based on this strain, a full-length HCV replicon was obtained and cloned in a plasmid, which replicon demonstrates an unusually high rate of replication of genomic RNA upon transfection of human hepatoma cells Huh-7 and the production of infectious virions into the culture medium [30, 31]. The resulting HCVcc infectious cultural virus was designated as clone JFH1 (genotype 2a). Next, a Huh-7 cell derivate was obtained, designated Huh-7.5, with a higher infectious potential and the ability to sustain prolonged infection of the JFH-1 clone HCV [32]. High permissivity of Huh-7.5 cells is owing to the presence of a mutation of the RIG-1 gene therein. The product of this gene is activated by viral dsRNA and is involved in the induction of interferon-mediated antiviral protection of the cell [33].

Evaluation of antiviral activity was performed by methods of polymerase chain reaction (hereinafter PCR) in real time and by immunohistochemistry. For detection of HCV, 6G7 mouse monoclonal antibodies against Core viral protein (FDA, USA) were used. The range of tested concentrations of PEG-IFN-λ was from 0,0085 to 8500 ng/ml of culture medium. The scheme of experiments included 3 consecutive stages:

1) preincubation of the monolayer of cells Huh7.5 PEG-IFN-λ;
2) infection of Huh7.5 cells with JFH1 virus in the absence of PEG-IFN-λ;
3) incubation with 10-fold dilutions of PEG-IFN-λ corresponding to the doses of preincubation.

Studies have shown that the 50 % inhibitory dose of PEG-IFN-λ lies in the concentration range of 0.0085-0.085 ng/ml, and at a concentration of 0.085 ng/ml PEG-IFN-λ already results in almost 80 % inhibition of HCV reproduction in human hepatomacells (Table 1).

Table 1. The level of inhibition of the hepatitis C virus reproduction by PEG-IFN-λ according to the results of real-time PCR

| The concentration of PEG-IFN-λ (expressed as IFN-λ), ng/ml | Inhibition rate of viral reproduction, % |
|---|---|
| 8,500.000 | 96.52 |
| 850.000 | 96.56 |
| 85.000 | 95.65 |
| 8.500 | 93.84 |
| 0.850 | 91.30 |
| 0.085 | 78.62 |

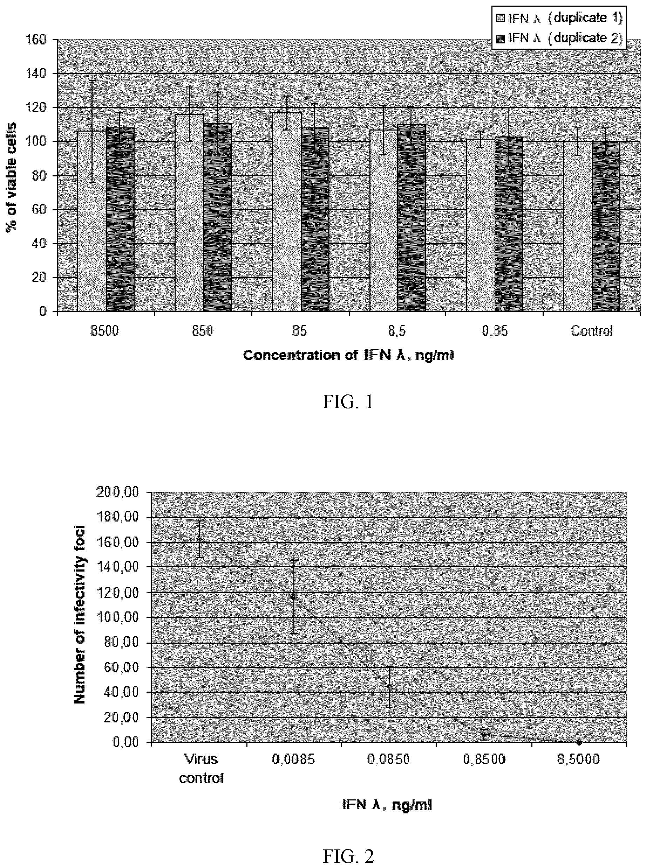

At the same time, the analysis of the cytotoxic activity of PEG-IFN-λ on human hepatoma cell culture Huh7.5 showed that even a dose of PEG-IFN-λ 8,500 ng/ml, which is a million times more than the effective antiviral dose, does not have any cytotoxic effect on Huh7.5 cells (Fig. 1). The method for measuring cytotoxicity used for the study is based on the ability of mitochondrial dehydrogenases to convert water-soluble 2,3-bis-(2-methoxy-4-nitro-5-sulfophenyl)-2H-tetrazolium-5-carboxanilide (XTT reagent, Sigma-Aldrich, USA) to formazan, wherein latter crystallizes inside the cell. Solubilization of formazan using phenazine metasulfate (PMS) and subsequent photometry make it possible to accurately correlate the change in the optical density of the solution with the change in the number of viable cells.

**Bioavailability of PEG-IFN-λ**

The study of the bioavailability of PEG-IFN-λ was carried out according to the "Guidelines for conducting pre-clinical studies of drugs" [34] on outbred laboratory rats weighing at least 180 g at the age of 12-14 weeks; animals were cared and manipulated in accordance with the SOP "Care and use of laboratory animals" and applicable international standards.

PEG-IFN-λ was administered to animals once at a dose of 2.6 μg/kg expressed asIFN-λ, intragastrically or intravenously, as a bolus. The blood serum IFN-λ concentration was determined when administered intravenously or intragastrically within the period of 0-24 hours after administration, and the IFN-λ concentration in the heart, omentum, liver, kidney, striated muscles and brain tissues of rats was determined within the same period of time after intragastric administration. Quantitative determination of IFN-λ in biological objects (blood serum, organs and tissues of animals) was performed using the enzyme immunoassay (ELISA) method. The experiments showed that the concentration of IFN-λ in blood serum when administered intravenously reached a maximum in 2 minutes after administration and amounted to 30947.83±1968.67 pg/ml. After intragastric administration, the concentration of IFN-λ in the blood serum reached a maximum in 4 hours after the administration and amounted to 1167.61±115.76 pg/ml. The maximum concentrations of IFN-λ in the tissues of rats after intragastric administration were: 1239.04±125.11 pg/g in the liver (in 2 hours after the administration), 975.29±109.23 pg/g in the kidneys (in 4 h after the administration), 488.11±81.53 pg/g in the heart (in2 hours after the administration), 392.92±37.78 pg/g in the striated muscles (in 8 hours after the administration). In the brain and omentum, the concentration of IFN-λ is below the threshold for determining (2 pg/mg) of the method used.

Calculation of pharmacokinetic parameters using the out-of-model statistical moments method [35] and the trapezoid method [36, 37] showed that with intragastric administration the maximum half-life, maximum concentration and minimum degree of elimination of IFN-λ were observed in rat liver and amounted to 10.11 h, 1.24 ng/g and 0.07 $h^{-1}$, respectively (Table 2).

Table 2 - Pharmacokinetic parameters of PEG-IFN-λ after a single intravenous and intragastric administration to rats at a dose of 2.6 μg/kg (expressed as IFN-λ) in blood serum and organs and tissues of rats

| | Intravenous administration | Intragastric administration | | | | |
|---|---|---|---|---|---|---|
| | Blood serum | Blood serum | Liver | Kidneys | Heart | Striated muscle |
| The total area under the experimental curve "concentration - time", AUC, (ng $\times$ h)/ml | 37.96 | 7.96 | 11.60 | 7.44 | 3.74 | 4.82 |
| Total area under the moment curve, AUMC, (ng $\times$ h$^2$)/ml | 52.33 | 51.40 | 166.68 | 62.54 | 32.30 | 47.90 |
| Maximum concentration, $C_{max}$ ng/ml, ng/g | 3.09 | 1.17 | 1.24 | 0.98 | 0.49 | 0.39 |
| Elimination rate, $k_{e1}$, hour-1 | 0.68 | 0.23 | 0.07 | 0.15 | 0.14 | 0.16 |
| Half-life, $T_{1/2}$, hour | 1.02 | 3.02 | 10.11 | 4.71 | 5.01 | 4.26 |

The absolute bioavailability (F) of PEG-IFN-λ was calculated by the formula:

$$F = \frac{AUCe/v \, x \, Di/v}{AUCi/v \, x \, De/v},$$

where:

$AUC_{e/v}$ is the area under the pharmacokinetic curve after extravascular administration;

$AUC_{i/v}$ is the area under the pharmacokinetic curve after intravenous administration;

$D_{i/v}$ is the dose level for intravenous administration;

$D_{e/v}$ is the dose level for extravascular administration.

The dose of PEG-IFN-λ administered to rats intravenously and intragastrically is the same, the area under the pharmacokinetic curve value for intravenous administration was 37.96 ng $\times$ h/ml, the area under the pharmacokinetic curve value for the extravascular route of administration was 7.96 ng $\times$ h/ml, therefore, the absolute bioavailability for PEG-IFN-λ was 20.97 % when administered orally.

Tissue bioavailability of PEG-IFN-λ was calculated by the formula:

$$ft = AUC_t / AUC_p,$$

where:

$AUC_t$ is the area under the pharmacokinetic curve of the substance in the tissue;
$AUC_p$ is the area under the serum pharmacokinetic curve.

Due to the fact that direct absorption of the therapeutic agent into the portal vein occurs after intragastric administration, the maximum tissue bioavailability was observed in the liver and amounted to 0.31; tissue bioavailability in the kidneys was 0.20; in the striated muscles 0.13; in the heart 0.10. At the same time, PEG-IFN-λ practically does not penetrate into the brain and does not accumulate in adipose tissue.

The difference between the proposed agent and the prototype and the technical results are as follows:

- the claimed agent has antiviral activity against hepatotropic viruses. Studies on the inhibition of HCV reproduction in human hepatoma cells showed that the 50 % inhibitory dose of PEG-IFN-λ lay in the concentration range of 0.0085-0.085 ng/ml, and at a concentration of 0.085 ng/ml PEG-IFN-λ resulted in almost 80 % inhibition of HCV reproduction;

- the claimed agent does not possess cytotoxic activity: even the dose of PEG-IFN-λ 8500 ng/ml, which is a million times more than the effective antiviral dose, does not have a cytotoxic effect on the human hepatoma cell culture Huh7.5;
- the absolute bioavailability of the claimed agent when administered orally is at least 10 %: the absolute bioavailability of PEG-IFN-λ after single intragastric administration to rats at a dose of 2.6 μg/kg expressed as IFN-λ is 20.97 %. This makes it possible to use the oral route of administration of the PEG-IFN-λ. medication, which eliminates the side effects common for the parenteral route of administration and increases the compliance of patients with therapy;
- the maximum tissue bioavailability of PEG-IFN-λ, the maximum concentration of IFN-λ, and the maximum half-life of IFN-λ after single intragastric administration to rats at a dose of 2.6 μg/kg expressed as IFN-λ are observed in the liver and amount to 0.31; 1239.04± 125.11 pg/g and 10.11 h, respectively. This is due to the direct absorption of PEG-IFN-λ into the portal vein and confirms the "targeted" effect of the claimed agent on the liver, thereby reducing the systemic side effects common for the parenteral route of administration;
- unlike the prototype obtained by chemical pegylation of IFN-λ, the claimed agent is obtained by pegylation of IFN-λ when the initial components are irradiated with ionizing radiation, which greatly simplifies the technological scheme of production and thereby reduces the cost of the final product, since it eliminates the multi-stage organic synthesis coupled with significant loss of the target protein substance at intermediate technological stages, and also the subsequent purification of the desired active molecules both from components, which did not enter into the reaction, and from excessively modified reaction products.

The claimed technical result is also achieved in a method for preparation of pegylated interferon lambda having antiviral activity against hepatotropic viruses and oral bioavailability of at least 10 %, which comprises preparing a solution of polyethylene glycol with a molecular weight from about 400 to about 5,000 Da and interferon lambda, freezing and cooling of the prepared solution to a temperature of -10 °C or below, and the subsequent irradiating of the frozen and cooled solution with ionizing radiation in a dose of from 0.1 to 0.5 Mrad, wherein the preparation of the solution of polyethylene glycol and interferon lambda is carried out by mixing a solution of interferon lambda and a solution of polyethylene glycol, and wherein the solution of polyethylene glycol is irradiated with ionizing radiation in a dose of 1 to 5 Mrad before mixing.

As polyethylene glycol with a molecular weight of from about 400 to about 5,000 Da, polyethylene glycol obtained by any known method is used, for example polyethylene glycol produced by OOO "Zavod syntanolov", Russia. Purified water or an aqueous salt buffer providing the maintenance of the pH of the solution in the limits required for the preservation of the protein later, preferably from 6.0 to 7.9, is used as a solvent for the preparation of the solution of polyethylene glycol, for example phosphate-saline buffer ($NaH_2PO_4$ 10 mmol/l, NaCl 150 mmol/l, pH= 7.4), and the concentration of PEG in the resulting solution ranges from 0.20 to 0.50 g/ml. The PEG solution is transferred into polyethylene bags or tubes in such a way that the thickness of the liquid layer does not exceed 5-8 mm, and sealed. As a container, it is possible to use any other package that is permeable to ionizing radiation and at the same time resistant to its effect. A beam of accelerated electrons in a dose of from 1 to 5 Mrad is used as an ionizing radiation.

The molecular weight of PEG and the dose of ionizing radiation were determined in preliminary experiments on the treatment of aqueous solutions of PEG with a molecular weight of from about 400 to about 20,000 Da with ionizing radiation in a dose of 0.25 to 10 Mrad, with further analysis of samples of irradiated PEG with gel-penetrating high-performance liquid chromatography using evaporative light scattering detector and chromato-mass-spectrometry. At present, it is known that irradiation of an aqueous solution of PEG with high doses of ionizing radiation leads to intermolecular cross-linking and the formation of hydrogels, regardless of its molecular weight. In particular, for 4 % aqueous solution of PEG with a molecular weight of 1,500 Da, the ability to form a hydrogel was detected when the absorbed dose of radiation reached 12-14 Mrad. At the same time, low molecular weight PEG, when irradiated in aqueous solutions at low doses and low concentrations of the polymer, has the ability to intramolecular cross-linking, leading to the synthesis of polymer coils, the so-called nanogels [38]. In the course of the experiments it was found that when exposed to ionizing radiation, PEG molecules undergo structural changes characterized by varying degrees of cross-linking, depending on both their molecular weight and the radiation dose. When a PEG solution with a molecular weight of 10,000 Da and higher is irradiated, even with a minimum absorbed dose of 0.25 Mrad, a significant aggregation of PEG molecules is observed with the formation of supramolecular structures of super-large masses $10^6$-$10^7$ Da. When irradiating solutions of PEG with a molecular weight of from about 400 to about 5,000 Da in a dose of 1 to 5 Mrad, characteristic intramolecular interactions that do not lead to pronounced pre-gelling and the formation of supramolecular associates are observed, which determines the choice of this type of polymers, since under these conditions PEG demonstrates a pronounced ability to intramolecular cross-linking at low energy costs and at the same time it forms a polymer matrix, which is later needed to incorporate the protein.

Natural or recombinant interferon lambda obtained by any known method is used as IFN-λ. To prepare a solution of IFN-λ, purified water or an aqueous salt buffer is used as a solvent to maintain the pH of the solution in the limits necessary for protein preservation, preferably from 6.0 to 7.9, for example phosphate-saline buffer (NaH$_2$PO$_4$ 10 mmol/l, NaCl 150 mmol/l, pH = 7.4). The concentration of interferon lambda in the resulting solution is 0.25 mg/ml or higher, preferably 0.9 mg/ml, but it should be sufficient so that when mixed with the PEG solution, the concentration of IFN-λ in the final mixture ranges from 0.2 to 0.8 mg/ml. It is also possible to use solutions of IFN-λ produced by manufacturers, for example interferon lambda 1 solution manufactured by ZAO «SCPhB», Russia.

Next, a mixture of irradiated PEG solution and IFN-λ solution is prepared. The use of IFN-λ in the form of a solution is necessary to provide uniform mixing, because when dissolved, dry IFN-λ requires intensive mixing, changing the viscosity and structure of the PEG solution. For the preparation of a mixture of the solutions of IFN-λ and PEG, the amounts of the solution of IFN-λ and the pre-irradiated PEG solution are selected so that after mixing the solutions, the concentration of IFN-λ in the resulting mixture ranges from 0.2 to 0.8 mg/ml, and the concentration of polyethylene glycol varies from 8 to 12 mg/ml, while water or an aqueous salt buffer providing maintenance of the pH of the solution in the limits necessary for protein preservation, preferably from 6.0 to 7.9, is used as a solvent. Optimal concentrations of IFN-λ and PEG were determined experimentally in order to achieve the highest preservation and stability of the protein and maintain the required excess of the polymer by weight relative to the protein substance content, since during the radiation exposure it is the excess of PEG molecules that first off faces main attack of free radicals, contributing to the increased preservation of the protein molecule. The resulting mixture is transferred to polyethylene bags or tubes in such a way that the thickness of the liquid layer does not exceed 5-8 mm, and sealed. Any other package permeable to ionizing radiation and at the same time resistant to effects thereof can be used as a container.

The packaged mixture is frozen and cooled to a temperature of -10 °C, wherein the mixture can be subjected to degassing in any available way before freezing. After that, the frozen and cooled mixture is irradiated with ionizing radiation in a frozen state, wherein abeam of accelerated electrons in a dose of 0.1 to 0.5 Mrad is used as ionizing radiation. Irradiation of the mixture in a frozen state is necessary to reduce the negative effect of the action of free radicals: with such irradiation of the mixture, the preservation of IFN-λ according to reverse phase high-performance liquid chromatography and SDS polyacrylamide gel electrophoresis reaches 69 %, while in the case of irradiating the same mixture without freezing, even with a minimum dose of 0.25 Mrad, the methods used reveal only residual amounts of IFN-λ. Carrying out the degassing of the mixture of the solutions before packaging and freezing also allows to increase the preservation of IFN-λ to 73-76 %. This is explained by the fact that the processes of the formation of free radicals in a polymer solution under the effect of ionizing radiation proceed with the participation of dissolved air oxygen, while degassing decreases the oxygen content in the solution. The values of the degree of binding of the protein into the polymer-protein conjugates in the degassed mixture and the untreated mixture differ slightly (N = 1.14 and N = 1.22, respectively), which means that the proportion of PEG chemically bound to interferon lambda, is about 0.4 % of its total amount. The bulk of PEG in the studied sample enters into non-covalent reversible interactions with protein molecules, with formation of supramolecular complexes.

After irradiation, the target product is stored frozen or subjected to defrosting and, if necessary, to additional purification using sterilizing filtration, after which, for convenience of storage, it is subjected to sterile dispensing to sterile package, for example glass vials.

The difference between the claimed method and the prototype and the technical results are that in the claimed method:

- solution of polyethylene glycol with a molecular weight of from about 400 to about 5,000 Da is used, which is subjected to irradiation with ionizing radiation before mixing with interferon lambda solution. When this occurs, structural changes emerge within the molecules of polyethylene glycol, which changes are not associated with molecular weight increasing, but lead to the formation of the polymer matrix necessary for protein incorporation;
- preparation of a solution of polyethylene glycol and interferon lambda is carried out by mixing a solution of interferon lambda and a solution of polyethylene glycol. This allows to avoid intensive mixing of the PEG solution required for dissolving dry interferon lambda, and at the same time to retain the viscosity and structure of the PEG solution necessary for effective pegylation of interferon lambda;
- the properties of interferon lambda are modified in such a way that its oral bioavailability is at least 10 %. At the same time, there is no formation of covalent bonds between interferon lambda and polyethylene glycol, which fact allows to avoid an uncontrolled change in the properties of interferon lambda and to use any available interferon as interferon lambda.

The invention is illustrated by the following examples of specific implementation.

**Example 1. PREPARATIONOF PEG-IFN-λ**

15.00 ml of purified water is added to 20.00 g of PEG-1500 (manufactured by OOO "Zavod synthanolov", Russia). The mixture is stirred on a magnetic stirrer until polyethylene glycol is completely dissolved, after which the volume of the mixture is adjusted to 50.00 ml. The resulting solution with a PEG-1500 concentration of 0.40 g/ml is transferred to a polyethylene bag in such a way that the thickness of the liquid layer does not exceed 5-8 mm, and sealed. The packaged PEG-1500 solution is exposed to ionizing radiation in a dose of 2.0 Mrad in the electron accelerator ИЛУ-10 (ЭУ-75).

80.00 ml of a solution of interferon lambda 1 (ZAO "SCPhB", Russia) with a concentration of interferon lambda 1 0.86 mg/ml is mixed with 3.45 ml of the irradiated solution of PEG-1500, the volume of the obtained mixture is adjusted to 138.00 ml with phosphate-saline buffer ($NaH_2PO_4$ 10 mmol/l, NaCl 150 mmol/l, pH = 7.4), and mixed thoroughly. Next, the degassing of the resulting solution is carried out using a vacuum pump and a Bunsen flask. Air is evacuated from the flask to a pressure of 10-50 mm Hg within 30-40 minutes. Then the degassed solution is placed toa polyethylene bag so that the liquid layer thickness in a horizontal position does not exceed 5-8 mm, the bag is sealed and frozen to -20 °C in a freezer for 12 hours, after which the irradiating with ionizing irradiation in a dose of 0.25 Mrad is carried out in an electron accelerator ИЛУ-10 (ЭУ-75). After irradiation, the package with PEG-IFN-λ1 is thawed, sterilizing filtration is carried out using "Stericup" vacuum sterilization system (Merck Millipore) followed by dispensing to sterile glass vials and sealing.

**Example 2. The study of the cytotoxic activity of PEG-IFN-λ**

The cytotoxic activity of PEG-IFN-λ1 obtained in Example 1 was determined on a human hepatoma cell culture Huh7.5 in the PEG-IFN-λ1 concentration range from 0.85 to 8500 ng/ml (expressed asIFN-λ1) by micromethod on 96-well plates ("Greiner") using 2,3-bis-(2-methoxy-4-nitro-5-sulfo-phenyl)-2H-tetrazolium-5-carboxanilide (XTT reagent, Sigma-Aldrich, USA). The method is based on the ability of mitochondrial dehydrogenases to convert water-soluble XTT to formazan, which crystallizes inside the cell. Solubilization of formazan using phenazine metasulfate (PMS) and subsequent photometry make it possible to accurately correlate the change in the optical density of the solution with the change in the number of viable cells. The specific cell death of Huh7.5 treated with different concentrations of cell proteins versus untreated (control, 100 % viability) was evaluated in duplicates. The results are presented in Fig. 1.

**Example 3. The study of the antiviral activity of PEG-IFN-λ**

Evaluation of the antiviral activity of PEG-IFN-λ1 obtained in Example 1 was carried out using the model of JFH1 clone/Huh-7.5 cells, which is currently the generally acknowledged system for assessing the antiviral activity of agents, including interferons.

Linearized DNA of the pJFH1 pUC plasmid was used as a template for transcription of full-length infectious RNA of HCV using the TranscriptAid™ reagent kit for highly efficient transcription with T7 RNA polymerase (Fermentas, Latvia).

Cultural hepatitis C virus (JFH1) was obtained by transfecting the synthesized RNA into human hepatoma cells Huh7.5 using the Mirus Bio MIR 2250 RNA transfection kit (Mirus, USA). The maximum yield of the virus to the culture medium after 8 passages of the transfected cells amounted to $1.37 \times 10^6$ copies of RNA/ml according to the results of real-time PCR ("АмплиСенс® HCV- Монитор-FL " test system, ILS, Moscow). Next, the virus-containing culture fluid was concentrated by centrifugation in a cell with shut-off filter Amicon Ultra-15 centrifugal filter unit (100 kDa) (Millipore, USA), and another 4 sub-passages were performed on Huh7.5 cells to obtain viral stock of JFH1. The stock was titrated on monolayer of Huh7.5 cells by the method of immunohistochemical determination of infectivity foci (hereinafter referred to as phi). For this, a monolayer of Huh7.5 cells grown in 96-well plates was infected with 10-fold dilutions of the JFH1 stock, incubated for 72 hours, washed with 1 x PBS, fixed with isopropanol, treated with 6G7 monoclonal antibodies against the Core 6G7 protein, and with secondary antibodies conjugated with horseradish peroxidase. The foci of infected cells were stained with AEC chromogen (3-Amino-9-ethylcarbazole, Sigma), which is oxidized by peroxidase to form an insoluble red precipitate. Foci scanning was performed using an "Axiovert" 40 CFL ZEISS microscope. The titer of HCV was $\sim 2 \times 10^4$ phi/ml. The resulting stock was dispensed in 1.5 μl aliquots and stored at - 80 °C until used for determining the antiviral activity of PEG-IFN-λ1.

To assess the antiviral activity of PEG-IFN-λ1 by real-time PCR, a monolayer of Huh7.5 cells grown on 24-well plates was incubated with 10-fold dilutions of PEG-IFN-λ1 in the range of 0.085-8500ng/ml in culture medium for 12 hours at 37 °C. Then the medium with PEG-IFN-λ1 was removed, and the cells were infected with the same dose 4.37 $\times 10^6$ copies of RNA/well (except for cell control)of JFH1 virus (from the packaged frozen stock), 3 wells per each dose

of PEG-IFN-$\lambda$1. Virus adsorption was carried out for 2 hours at 37 °C without PEG-IFN-$\lambda$1, then the virus was removed and the medium with the corresponding dilutions of PEG-IFN-$\lambda$1 was added to the wells again. The plates were incubated for 2 days at 37 °C in a $CO_2$ incubator, then the lysates of infected and control cells were prepared as follows:

- the cell monolayer was washed three times with TBS buffer (150 mM NaCl, 50 mM Tris, pH 7.6);
- treated with a mixture of lysis buffer 100 $\mu$l/well (150 mM NaCl, 50 mM Tris, p 7.6, 1 % Triton X-100) and protease inhibitors cocktail (AEBSF, aprotinin, bestatin, E-64, EDTA, leupeptin) (Sigma, USA), transferred to microcentrifuge tubes, kept on ice for 10 minutes, and frozen;
- lysates were frozen and thawed three times;
- the resulting mixture was centrifuged for 30 minutes at a centrifugation rate of 14,000 rpm and a temperature of 4 °C;
- the supernatant was divided in 100 $\mu$l aliquots, and PCR was performed in real time using the "АмплиСенс® HCV-Монитор-FL" test system.

The results of the PCR are presented in Table 3.

Table 3. The level of inhibition of reproduction of the hepatitis C virus by PEG-IFN-$\lambda$1 according to the results of real-time PCR

| Sample name (cell lysates) | Concentration of PEG-IFN-$\lambda$1 (expressed asIFN-$\lambda$1), ng/ml | PCR result, IU/ml | Concentration of virus in the sample, copies of RNA/ml |
|---|---|---|---|
| Lys CC * | - | $2.86 \times 10^3$ | $5.72 \times 10^3$ |
| 0 | 8,500.000 | $9.6 \times 10^5$ | $1.92 \times 10^6$ |
| 1 | 850.000 | $9.5 \times 10^5$ | $1.90 \times 10^6$ |
| 2 | 85.000 | $1.2 \times 10^6$ | $2.4 \times 10^6$ |
| 3 | 8.500 | $1.7 \times 10^6$ | $3.4 \times 10^6$ |
| 4 | 0.850 | $2.4 \times 10^6$ | $4.8 \times 10^6$ |
| 5 | 0.085 | $5.88 \times 10^6$ | $1.18 \times 10^7$ |
| Lys CV ** | - | $2.76 \times 10^7$ | $5.52 \times 10^7$ |
| * - lysate of non-infected cells Huh7.5, negative control (blank); ** - lysate of Huh7.5 cells infected with the virus, but not treated with IFN-$\lambda$1, positive control, 100 % infection. | | | |

Evaluation of the antiviral effect of PEG-IFN-$\lambda$1 was also performed by immunohistochemical method of counting the infectivity foci, described earlier, on a monolayer of Huh 7.5 cells in the range of PEG-IFN-$\lambda$1 concentrations from 0.0085 to 8.5000 ng/ml. The results of this analysis are presented in Fig. 2, where it can be seen that the dose of PEG-IFN-$\lambda$1 0.0085 ng/ml inhibits the reproduction of the virus by 25 %, and the next 10-fold dose of 0.085 ng/ml already by 75 %, thus the 50 % inhibitory dose lies in the range of concentrations of PEG-IFN-$\lambda$1 of 0,0085-0,085 ng/ml.

**Example 4. Study of the bioavailability of PEG-IFN-$\lambda$**

A study of the bioavailability of PEG-IFN-$\lambda$1 obtained in Example 1 was carried out according to the "Guidelines for conducting pre-clinical studies of drugs"[34]. As test systems, outbred laboratory rats weighing at least 180 g at the age of 12-14 weeks were used. They were obtained from the Department of Experimental Biological Models of the SRIPh&RM named after E.D. Goldberg of the Federal State Budgetary Scientific Institution "Tomsk National Research Medical Center of the Russian Academy of Sciences" (veterinary certificates are available); animals were cared and manipulated in accordance with the SOP "Care of laboratory animals" and international standards in force at the moment (Animal Welfare Act, Guide for Care and Use of Laboratory Animals).

PEG-IFN-$\lambda$1 was administered to animals once at a dose of 2.6 $\mu$g/kg expressed as IFN-$\lambda$1 intragastrically or intravenously. The concentration of IFN-$\lambda$1 after a single intragastric administration of PEG-IFN-$\lambda$1 was determined in blood serum, as well as in the tissues of the heart, omentum, liver, kidneys, striated muscles and brain of rats prior to the administration of the drug and in 0.25, 0.5, 1, 2, 4, 8, 16 and 24 hours after administration; after a single intravenous administration: in the blood serum prior to the administration of the drug and in 0.03, 0.17, 0.33, 0.5, 0.75, 1 and 2 hours after the administration. 6 animals were used at each point.

Preliminary sample preparation was carried out as follows:

Blood serum. Blood was taken from the heart cavity after killing the animal. To separate the serum from the clot of cellular elements, the collected blood was circled along the walls of the tube with a glass rod, incubated at 37 °C for 30 minutes, kept in the refrigerator for 1 hour, and centrifuged at 3,000 rpm for 10 min. Isolated serum was collected in plastic tubes and frozen at a temperature of from - 18 to -20 °C. As a zero point (prior to the administration), serum samples of 6 intact animals obtained in a similar way were used.

Tissue of the liver, brain, heart, skeletal (striated) muscle, kidneys, omentum and small intestine. To measure IFN-$\lambda$1 in organs and tissues, a weighted amount of the tissue (about 1 g) was frozen and thawed twice, carefully ground in a mortar, and 20 % homogenates were prepared in distilled water. The resulting suspension was shaken on a shaker for 10 min. After that, the tubes were centrifuged at 3,000 rpm for 10 min. The supernatant was transferred to clean tubes and used for analysis. As a zero point (prior to the administration), samples of liver, brain, heart, skeletal muscle, kidney and omentum tissue of 6 intact animals obtained in a similar way were used.

Quantitative determination of IFN-$\lambda$1 in biological objects (blood serum, organs and tissues of animals) was performed using an enzyme immunoassay (ELISA) method using Human IL-29 Platinum ELISA kits ("Bender MedSystems GmbH", Austria, cat. No. BMS2049) designed to determine the concentration of human IFN-$\lambda$1 in culture supernatants, serum, plasma and other biological samples in the concentration range of from 2 to 1,000 pg/ml, in accordance with the manufacturer's instructions. The results were recorded on the immunoenzyme reactions analyzer АИФР-01 "УНИПЛАН" (ZAO "Pikon", Russia) at a wavelength of 450 nm.

The results of determining the concentrations of IFN-$\lambda$1 in the blood serum and organs and tissues of rats after a single intragastric administration of PEG-IFN-$\lambda$1 are presented in Table 4. Data on the quantitative determination of the concentration of IFN-$\lambda$1 in the brain and omentum are not presented, since the concentration was below 2 pg/ml and was not detected by ELISA using Human IL-29 Platinum ELISA kits.

The results of determining the concentrations of IFN-$\lambda$1 in the blood serum of rats after a single intravenous administration of PEG-IFN-$\lambda$1 at a dose of 2.6 $\mu$g/kg are presented in Table 5.

Calculation of pharmacokinetic parameters based on the data obtained was carried out using the out-of-model statistical moments method [39] and the trapezoidal method [40, 41]. The calculation results are presented in Table 2.

Table 4 - The concentration of IFN-$\lambda$ in the blood serum and organs and tissues of rats after a single intragastric administration of PEG-IFN-$\lambda$1 at a dose of 2.6 $\mu$g/kg (expressed asIFN-$\lambda$1)

| Time , hours | Concentration of IFN-$\lambda$ in blood serum, pg/ml | Concentration of IFN-$\lambda$1 in tissues, pg/g of tissue | | | |
|---|---|---|---|---|---|
| | | liver | kidney | heart | striated muscles |
| 0.25 | 28.93±4.37 | 37.66±6.05 | 18.20±3.05 | 11.62±1.86 | 11.61±1.84 |
| 0.5 | 74.16±11.82 | 84.52±10.25 | 57.39±7.81 | 33.92±6.82 | 37.98±5.55 |
| 1 | 212.41±38.97 | 329.01±35.29 | 162.08±25.29 | 124.36±14.7 3 | 122.85±18.6 4 |
| 2 | 503.02±71.06 | 1239.04±125.1 1 | 410.36±52.18 | 488.11±81.5 3 | 185.19±21.8 1 |
| 4 | 1167.61±115.7 6 | 734.09±76.72 | 975.29±109.2 3 | 258.06±33.7 9 | 232.63±33.9 1 |
| 8 | 445.33±61.03 | 396.07±50.65 | 338.88±51.71 | 189.90±23.60 0 | 392.92±37.7 8 |
| 16 | 92.18±12.66 | 239.14±33.94 | 157.76+29.49 | 98.35±17.98 | 132.87±22.8 6 |
| 24 | 10.91±1.83 | 172.84±27.85 | 41.56+13.30 | 14.51±3.82 | 28.84±3.48 |

Table 5 - The concentration of IFN-$\lambda$1 in the blood serum of rats after a single intravenous injection of PEG-IFN-$\lambda$1 at a dose of 2.6 $\mu$g/kg (expressed as IFN-$\lambda$1)

| Time, hours | Concentration of IFN-$\lambda$1 in blood serum, pg/ml |
|---|---|
| 0.00 | 0.00±0.00 |
| 0.03 | 30947.83±1968.67 |
| 0.17 | 24314.77±2362.67 |
| 0.33 | 21547.21±1828.17 |
| 0.50 | 18502.97±2143.13 |

(continued)

| Time, hours | Concentration of IFN-$\lambda$1 in blood serum, pg/ml |
|---|---|
| 0.75 | 14786.24±1323.60 |
| 1.00 | 11187.96±1057.21 |
| 2.00 | 6495.04±1062.71 |

**Literature sources**

1. Perez-Martin E, Weiss M, Diaz-San Segundo F, Pacheco JM, Arzt J, Grubman MJ, de los Santos T. Bovine type III interferon significantly delays and reduces the severity of foot-and-mouth disease in cattle. // J Virol. - 2012 - Vol. 86, №8. - P. 4477-4487.

2. Prokunina-Olsson L, Muchmore B, Tang W, Pfeiffer RM, Park H, Dickensheets H, Hergott D, Porter-Gill P, Mumy A, Kohaar I, Chen S, Brand N, Tarway M, Liu L, Sheikh F, Astemborski J, Bonkovsky HL, Edlin BR, Howell CD, Morgan TR, Thomas DL, Rehermann B, Donnelly RP, O'Brien TR. A variant upstream of IFNL3 (IL28B) creating a new interferon gene IFNL4 is associated with impaired clearance of hepatitis C virus. // Nat Genet. - 2013. - Vol.45(2). - P. 164-71.

3. O'Brien TR, Prokunina-Olsson L, Donnelly RP. IFN-$\lambda$4: The Paradoxical New Member of the Interferon Lambda Family. // J Interferon Cytokine Res. - 2014. - Vol. 34(11). - P. 829-838.

4. Grigoryan S.S. Interferons lambda (type 3 interferons) and viral infections. // Interferon-2011 / Collection of scientific articles. - Moscow. - 2012. - 512 p. P. 63-72

5. Durbin, R.K., Kotenko, S.V., and Durbin, J.E. Interferon induction and function at the mucosal surface. // Immunol. Rev. - 2013. - Vol. 255. - P. 25-39.

6. Hermant, P., and Michiels, T. Interferon-$\lambda$ in the context of viral infections: production, response and therapeutic implications. // J. Innate Immun. - 2014. - Vol. 6. - P. 563-574.

7. Mahlakoiv, T., Hernandez, P., Gronke, K., Diefenbach, A., and Staeheli, P. Leukocyte-derived IFN-a/b and epithelial IFN-1 constitute a compartmentalized mucosal defense system that restricts enteric virus infections. // PLoS Pathog. - 2015. - Vol. 11. - art. no. e1004782.

8. Ank N, West H, Bartholdy C, Eriksson K, Thomsen AR, Paludan SR. Lambda interferon (IFN-lambda), a type III IFN, is induced by viruses and IFNs and displays potent antiviral activity against select virus infections in vivo. // J Virol. - 2006. - Vol. 80(9). - P. 4501-4509.

9. Mordstein, M., Neugebauer, E., Ditt, V., Jessen, B., Rieger, T., Falcone, V., Sorgeloos, F., Ehl, S., Mayer, D., Kochs, G., et al. Lambda interferon renders epithelial cells of the respiratory and gastrointestinal tracts resistant to viral infections. // J. Virol. - 2010. - Vol. 84. - P. 5670-5677.

10. Pott J, Mahlakõiv T, Mordstein M, Duerr CU, Michiels T, Stockinger S, Staeheli P, Hornef MW. IFN-lambda determines the intestinal epithelial antiviral host defense. // Proc Natl Acad Sci USA. - 2011. - Vol. 108(19). - P. 7944-7949.

11. Heinze B, Frey S, Mordstein M, Schmitt-Gräff A, Ehl S, Buchholz UJ, Collins PL, Staeheli P, Krempl CD. Both nonstructural proteins NS1 and NS2 of pneumonia virus of mice are inhibitors of the interferon type I and type III responses in vivo. // J Virol. 2011 May;85(9):4071-84. doi: 10.1128/JVI.01365-10. Epub 2011 Feb 9.

12. Nice, T.J., Baldridge, M.T., McCune, B.T., Norman, J.M., Lazear, H.M., Artyomov, M., Diamond, M.S., Virgin, H.W. Interferon-$\lambda$ cures persistent murine norovirus infection in the absence of adaptive immunity. // Science. - 2015. - Vol. 347(6219). - P. 269-273.

13. Lazear, H.M., Daniels, B.P., Pinto, A.K., Huang, A.C., Vick, S.C., Doyle, S.E., Gale, M., Jr., Klein, R.S., and Diamond, M.S. Interferon-1 restricts West Nile virus neuroinvasion by tightening the blood-brain barrier. // Sci. Transl. Med. - 2015. - Vol. 7. - P. 284ra59.

14. Thatte A, DeWitte-Orr SJ, Lichty B, Mossman KL, Ashkar AA. A critical role for IL-15 in TLR-mediated innate antiviral immunity against genital HSV-2 infection. // Immunol Cell Biol. - 2011. - Vol. 89(6). - P. 663-669.

15. Bartlett NW, Buttigieg K, Kotenko SV, Smith GL. Murine interferon lambdas (type III interferons) exhibit potent antiviral activity in vivo in a poxvirus infection model. // J Gen Virol. - 2005. - Vol. 86(Pt 6). - P. 1589-1596.

16. Pagliaccetti N.E., Chu E.N., Bolen Ch.R., Kleinstein S.H., Robek M.D. Lambda and alpha interferons inhibit hepatitis B virus replication through a common molecular mechanism but with different in vivo activities. // Virology. - 2010.- Vol. 401(2). - P. 197-206.

17. Donnelly, R.P., Dickensheets, H., and O'Brien, T.R. Interferon-lambda and therapy for chronic hepatitis C virus infection. // Trends Immunol. - 2011. - Vol. 32. - P. 443-450.

18. Hayes, C.N., Imamura, M., Aikata, H., and Chayama, K. Genetics of IL28B and HCV-response to infection and treatment. // Nat. Rev. Gastroenterol. Hepatol. - 2012. - Vol. 9. - P. 406-417.

19. Global health sector strategy on viral hepatitis 2016-2021. Towards elimination of viral hepatitis. - WHO, Geneva, Switzerland, 2016. - 56 p.

20. New recommendations in the updated WHO Guidelines for screening, care and treatment of persons with chronic hepatitis C infection. Analytical reference. - WHO, Geneva, Switzerland, 2016. - 12 p.

21. Patent US 5951974, МПк A61P43/00, A61P35/02, A61K47/34, A61P1/16, C07K1/08, C07K14/56, A61P35/00, A61K38/21, C07K1/113, A61P31/12, A61K47/48, A61K38/00, publ. 14.09.1999.

22. Patent EP 0 809 996, МПк C07K1/10, C07K14/56, A61P35/00, A61P37/02, A61K31/00, A61P31/00, A61P31/12, A61K31/745, C07K14/52, C07K14/555, C07K1/113, A61K47/48, A61K38/21, A61P37/00, C07K17/08, A61P31/04, publ. 03.12.1997.

23. Patent RU 2 447 083, МПк C07K 14/56, A61K 38/21, A61K 47/48, A61P 37/00, A61P 35/00, A61P 31/12, publ. 10.04.2012.

24. Patten S.B. What is the best approach to treating interferon-induced depression in people with multiple sclerosis? // J. Neurosci. - 2001.- V. 26. - P. 66.

25. Wills R.J. Clinical pharmacokinetics of interferons // Clin. Pharmacokinet. - 1990. - Vol.19. - № 5. - P. 390-399.

26. Prummer O., Streichan U., Porzsolt F. Treatment-induced interferon (IFN)-$\alpha$ antibodies: Differential neutralisation of the antiviral and antiproliferative IFN-$\alpha$ activity in vitro // J. Interferon Res. - 1991. - № 11. - P. 265.

27. Muir, A.J., Arora, S., Everson, G., Flisiak, R., George, J., Ghalib, R., Gordon, S.C., Gray, T., Greenbloom, S., Hassanein, T., et al.; EMERGE study group A randomized phase 2b study of peginterferon lambda-la for the treatment of chronic HCV infection. // J. Hepatol. - 2014. - Vol. 61. - P. 1238-1246.

28. Patent RU 2496514, МПк A61K 38/21, A61K 38/20, A61P 31/20, publ. 27.10.2013.

29. Patent RU 2409669, МПк C12N 11/08, C12N 11/10, A61K 38/00, A61K 38/43, publ. 27.05.2012.

30. Kato T., Date T., Murayama A. et al. Cell culture and infection system for hepatitis C virus. // Nature protocols - 2006. - Vol. 1(5). - P.2334-2339.

31. Kato N., Mori K., Abe K. et al. Efficient replication systems for hepatitis C virus using a new human hepatoma cell line. // Virus Research. - 2009. - Vol. 146. - P.41-50.

32. Blight K.J., McKeating J.A., Rice C.M. Highly permissive cell lines for subgenomic and genomic hepatitis C virus RNA replication. // J. Virol. - 2002. - Vol. 76(24). - P.13001-13014.

33. Bartenschlager R. and Pietschmann T. Efficient hepatitis C virus cell culture system: What a difference the host cell makes. // PNAS. - 2005. - Vol. 102(28). - P.9739-9740.

34. Guidelines for conducting preclinical studies of drugs. Part one. - M .: Grief and K, 2013. -944 p.

35. Piotrovsky V.K. The method of statistical moments and integral model-independent parameters of pharmacokinetics // Pharmacology and Toxicology. - 1986. - № 5. - P. 118-125.

36. Karkishchenko N.N., Khoronko V.V., Sergeeva S.A., Karkishchenko V.N. Pharmacokinetics. - Rostov-on-Don, 2001. - 383 p.

37. Miroshnichenko I.I. Basics of pharmacokinetics. - M .: Publishing house "GEOTAR-MED", 2002. - 188 p.

38. Rosiak J. M., Janik I., Kadlubowski S., Kozicki M.,Kujawa P., Stasica P., Ulanski P. Radiation formation of hydrogels for biomedical application // Radiation synthesis and modification of polymers for biomedical applications Final results of a coordinated research project 1996-2000. - 2002. - P. 5-48.

## Claims

1. Pegylated interferon lambda having antiviral activity against hepatotropic viruses, wherein said interferon has oral bioavailability at least 10% and is obtained by irradiating a solution of polyethylene glycol with a molecular weight of from about 400 to about 5,000 Da by ionizing radiation in a dose of 1 to 5 Mrad, mixing the resulting solution with an interferon lambda solution, freezing and cooling of the obtained mixture to a temperature of -10 °C or below, and irradiating the frozen and cooled mixture with ionizing radiation in a dose of 0.1 to 0.5 Mrad.

2. A method for preparation of pegylated interferon lambda according to claim 1, comprising preparing a solution of polyethylene glycol with a molecular weight of from about 400 to about 5,000 Da and interferon lambda, freezing and cooling of the obtained solution to -10 °C or below, and subsequent irradiating of the frozen and cooled solution with ionizing radiation in a dose of 0.1 to 0.5 Mrad, wherein the preparation of the solution of polyethylene glycol and interferon lambda is carried out by mixing a solution of interferon lambda and a solution of polyethylene glycol, wherein the polyethylene glycol solution is irradiated with ionizing radiation in a dose of from 1 to 5 Mrad before mixing.

3. The method according to claim 2, wherein purified water or an aqueous salt buffer providing the maintenance of the pH of the solution in the limits necessary for the protein preservation, preferably from 6.0 to 7.9, is used as a solvent to prepare the solution of polyethylene glycol, and the concentration of polyethylene glycol in the resulting solution is from 0.20 to 0.50 g/ml.

4. The method according to claim 2, wherein natural or recombinant interferon lambda obtained by any known method is used as interferon lambda.

5. The method according to claim 2, wherein purified water or an aqueous salt buffer providing the maintenance of the pH of the solution in the limits necessary for the protein preservation, preferably from 6.0 to 7.9, is used as a solvent to prepare the solution of interferon lambda, and the concentration of interferon lambda in the resulting solution is 0.25 mg/ml or higher, preferably 0.9 mg/ml.

6. The method according to claim 2, wherein the amounts of the solution of interferon lambda and the pre-irradiated solution of polyethylene glycol are selected so that after mixing the solutions, the concentration of interferon lambda in the resulting mixture ranges from 0.2 to 0.8 mg/ml, and the concentration of polyethylene glycol is from 8 to 12 mg/ml, while purified water or an aqueous salt buffer providing the maintenance of the pH of the solution in the limits necessary for the protein preservation, preferably from 6.0 to 7.9, is used as a solvent.

7. The method according to claim 2, wherein the mixture of the interferon lambda solution and the pre-irradiated solution of polyethylene glycol is subjected to degassing in any available way before freezing.

8. The method according to claim 2, wherein the desired product is additionally subjected to final purification by sterilizing filtration.

FIG. 1

FIG. 2

## INTERNATIONAL SEARCH REPORT

| | International application No. |
|---|---|
| | PCT/RU 2018/050082 |

**A. CLASSIFICATION OF SUBJECT MATTER**

please see extra sheet

According to International Patent Classification (IPC) or to both national classification and IPC

**B. FIELDS SEARCHED**

Minimum documentation searched (classification system followed by classification symbols)

A61K,A61P,B01J

Documentation searched other than minimum documentation to the extent that such documents are included in the fields searched

Electronic data base consulted during the international search (name of data base and, where practicable, search terms used)

PatSearch (RUPTO internal), Espacenet, DWPI, PAJ, USPTO, CIPO, DEPATIS, EAPO

**C. DOCUMENTS CONSIDERED TO BE RELEVANT**

| Category* | Citation of document, with indication, where appropriate, of the relevant passages | Relevant to claim No. |
|---|---|---|
| Y | RU 2496514 C2 (ZAIMODZHINETIKS, INK. et al.) 27.10.2013, the abstract, p.12, lines 1-5, p.22, lines 3, 9-11, p.35, lines 46-51 | 1-8 |
| Y | RU 2409669 C2 (OOO 'SAENTIFIK FJUCHER MENEDZHMENT') 20.01.2011, the abstract, p.5, lines 43-47, p.10, lines 25-50, p. 11, lines 25-28, p.15, lines 48-51 | 1-8 |
| Y | RU 2554761 C1 (ZAKRYTOE AKTSIONERNOE OBSCHESTVO 'SIBIRSKY TSENTR FARMAKOLOGII I BIOTEKHNOLOGII') 27.06.2015, the abstract, p.4, lines 18-23 | 1-8 |
| Y | KINSHT D.N. et al. Spetsificheskaya protivovirusnaya aktivnost v otnoshenii virusa gepatita S peroralnogo lekarstvennogo preparata na osnove immobilizovannogo interferona lyambda-1 // XI mezh-dunarodny nauchny kongress «Ratsionalnaya farmakoterapiya»: sbornik nauchnykh materialov, izd. SPbGEU, 2016, p.81-83 | 1-8 |
| Y | KUBETZKO S. et al. Protein PEGylation Decreases Observed Target Association Rates via a Dual Blocking Mechanism // Mol. Pharmacol., 2005, V.68, p.1441, col.1, para.2 | 1-8 |
| A | TIAN S. et al. Suppression of hepatocellular carcinoma proliferation and hepatitis B surface antigen secretion with interferon-λ1 or PEG-interferon-λ1. The FASEB Journal, 2014, V.28, pp.3528-2539 | 1-8 |

☐ Further documents are listed in the continuation of Box C.    ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 06 November 2018 (06.11.2018) | 08 November 2018 (08.11.2018) |

| Name and mailing address of the ISA/   RU | Authorized officer |
|---|---|
| Facsimile No. | Telephone No. |

Form PCT/ISA/210 (second sheet) (July 1998)

INTERNATIONAL SEARCH REPORT

International application No.

CLASSIFICATION OF SUBJECT MATTER

PCT/RU 2018/050082

*A61K 38/21* (2006.01)
*A61K 47/60* (2017.01)
*A61P 31/12* (2006.01)
*B01J 19/08* (2006.01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5951974 A
- EP 0809996 A
- RU 2447083

- RU 2496514
- RU 2409669


**Non-patent literature cited in the description**

- **PEREZ-MARTIN E ; WEISS M ; DIAZ-SAN SEGUNDO F ; PACHECO JM ; ARZT J ; GRUBMAN MJ ; DE LOS SANTOS T.** Bovine type III interferon significantly delays and reduces the severity of foot-and-mouth disease in cattle. *J Virol.,* 2012, vol. 86 (8), 4477-4487
- **PROKUNINA-OLSSON L ; MUCHMORE B ; TANG W ; PFEIFFER RM ; PARK H ; DICKENSHEETS H ; HERGOTT D ; PORTER-GILL P ; MUMY A ; KOHAAR I.** A variant upstream of IFNL3 (IL28B) creating a new interferon gene IFNL4 is associated with impaired clearance of hepatitis C virus. *Nat Genet.,* 2013, vol. 45 (2), 164-71
- **O'BRIEN TR ; PROKUNINA-OLSSON L ; DONNELLY RP.** IFN-λ4: The Paradoxical New Member of the Interferon Lambda Family. *J Interferon Cytokine Res.,* 2014, vol. 34 (11), 829-838
- **GRIGORYAN S.S.** Interferons lambda (type 3 interferons) and viral infections. *Interferon-2011 / Collection of scientific articles,* 2012, vol. 512, 63-72
- **DURBIN, R.K. ; KOTENKO, S.V. ; DURBIN, J.E.** Interferon induction and function at the mucosal surface. *Immunol. Rev.,* 2013, vol. 255, 25-39
- **HERMANT, P. ; MICHIELS, T.** Interferon-λ in the context of viral infections: production, response and therapeutic implications. *J. Innate Immun.,* 2014, vol. 6, 563-574
- **MAHLAKOIV, T. ; HERNANDEZ, P. ; GRONKE, K. ; DIEFENBACH, A. ; STAEHELI, P.** Leukocyte-derived IFN-a/b and epithelial IFN-1 constitute a compartmentalized mucosal defense system that restricts enteric virus infections. *PLoS Pathog,* 2015, vol. 11
- **ANK N ; WEST H ; BARTHOLDY C ; ERIKSSON K ; THOMSEN AR ; PALUDAN SR.** Lambda interferon (IFN-lambda), a type III IFN, is induced by viruses and IFNs and displays potent antiviral activity against select virus infections in vivo. *J Virol.,* 2006, vol. 80 (9), 4501-4509

- **MORDSTEIN, M. ; NEUGEBAUER, E. ; DITT, V. ; JESSEN, B. ; RIEGER, T. ; FALCONE, V. ; SORGELOOS, F. ; EHL, S. ; MAYER, D. ; KOCHS, G. et al.** Lambda interferon renders epithelial cells of the respiratory and gastrointestinal tracts resistant to viral infections. *J. Virol.,* 2010, vol. 84, 5670-5677
- **POTT J ; MAHLAKÕIV T ; MORDSTEIN M ; DUERR CU ; MICHIELS T ; STOCKINGER S ; STAEHELI P ; HORNEF MW.** IFN-lambda determines the intestinal epithelial antiviral host defense. *Proc Natl Acad Sci USA.,* 2011, vol. 108 (19), 7944-7949
- **HEINZE B ; FREY S ; MORDSTEIN M ; SCHMITT-GRÄFF A ; EHL S ; BUCHHOLZ UJ ; COLLINS PL ; STAEHELI P ; KREMPL CD.** Both non-structural proteins NS1 and NS2 of pneumonia virus of mice are inhibitors of the interferon type I and type III responses in vivo. *J Virol.,* May 2011, vol. 85 (9), 4071-84
- **NICE, T.J. ; BALDRIDGE, M.T. ; MCCUNE, B.T. ; NORMAN, J.M. ; LAZEAR, H.M. ; ARTYOMOV, M. ; DIAMOND, M.S. ; VIRGIN, H.W.** Interferon-λ cures persistent murine norovirus infection in the absence of adaptive immunity. *Science,* 2015, vol. 347 (6219), 269-273
- **LAZEAR, H.M. ; DANIELS, B.P. ; PINTO, A.K. ; HUANG, A.C. ; VICK, S.C. ; DOYLE, S.E. ; GALE, M., JR. ; KLEIN, R.S. ; DIAMOND, M.S.** Interferon-1 restricts West Nile virus neuroinvasion by tightening the blood-brain barrier. *Sci. Transl. Med.,* 2015, vol. 7, 284ra59
- **THATTE A ; DEWITTE-ORR SJ ; LICHTY B ; MOSSMAN KL ; ASHKAR AA.** A critical role for IL-15 in TLR-mediated innate antiviral immunity against genital HSV-2 infection. *Immunol Cell Biol.,* 2011, vol. 89 (6), 663-669
- **BARTLETT NW ; BUTTIGIEG K ; KOTENKO SV ; SMITH GL.** Murine interferon lambdas (type III interferons) exhibit potent antiviral activity in vivo in a poxvirus infection model. *J Gen Virol.,* 2005, vol. 86, 1589-1596

- **PAGLIACCETTI N.E. ; CHU E.N. ; BOLEN CH.R. ; KLEINSTEIN S.H. ; ROBEK M.D.** Lambda and alpha interferons inhibit hepatitis B virus replication through a common molecular mechanism but with different in vivo activities. *Virology,* 2010, vol. 401 (2), 197-206
- **DONNELLY, R.P. ; DICKENSHEETS, H. ; O'BRIEN, T.R.** Interferon-lambda and therapy for chronic hepatitis C virus infection. *Trends Immunol.,* 2011, vol. 32, 443-450
- **HAYES, C.N. ; IMAMURA, M. ; AIKATA, H. ; CHAYAMA, K.** Genetics of IL28B and HCV-response to infection and treatment. *Nat. Rev. Gastroenterol. Hepatol.,* 2012, vol. 9, 406-417
- Global health sector strategy on viral hepatitis 2016-2021. *Towards elimination of viral hepatitis. - WHO,* 2016, 56
- New recommendations in the updated WHO Guidelines for screening, care and treatment of persons with chronic hepatitis C infection. *Analytical reference. - WHO,* 2016, 12
- **PATTEN S.B.** What is the best approach to treating interferon-induced depression in people with multiple sclerosis?. *J. Neurosci.,* 2001, vol. 26, 66
- **WILLS R.J.** Clinical pharmacokinetics of interferons. *Clin. Pharmacokinet.,* 1990, vol. 19 (5), 390-399
- **PRUMMER O. ; STREICHAN U. ; PORZSOLT F.** Treatment-induced interferon (IFN)-$\alpha$ antibodies: Differential neutralisation of the antiviral and antiproliferative IFN-$\alpha$ activity in vitro. *J. Interferon Res.,* 1991, (11), 265
- **MUIR, A.J. ; ARORA, S. ; EVERSON, G. ; FLISIAK, R. ; GEORGE, J. ; GHALIB, R. ; GORDON, S.C. ; GRAY, T. ; GREENBLOOM, S. ; HASSANEIN, T. et al.** EMERGE study group A randomized phase 2b study of peginterferon lambda-la for the treatment of chronic HCV infection. *J. Hepatol.,* 2014, vol. 61, 1238-1246
- **KATO T. ; DATE T. ; MURAYAMA A. et al.** Cell culture and infection system for hepatitis C virus. *Nature protocols,* 2006, vol. 1 (5), 2334-2339
- **KATO N. ; MORI K. ; ABE K. et al.** Efficient replication systems for hepatitis C virus using a new human hepatoma cell line. *Virus Research,* 2009, vol. 146, 41-50
- **BLIGHT K.J. ; MCKEATING J.A. ; RICE C.M.** Highly permissive cell lines for subgenomic and genomic hepatitis C virus RNA replication. *J. Virol.,* 2002, vol. 76 (24), 13001-13014
- **BARTENSCHLAGER R. ; PIETSCHMANN T.** Efficient hepatitis C virus cell culture system: What a difference the host cell makes. *PNAS,* 2005, vol. 102 (28), 9739-9740
- Guidelines for conducting preclinical studies of drugs. Part one. *M .: Grief and K,* 2013, 944
- **PIOTROVSKY V.K.** The method of statistical moments and integral model-independent parameters of pharmacokinetics. *Pharmacology and Toxicology,* 1986, (5), 118-125
- **KARKISHCHENKO N.N. ; KHORONKO V.V. ; SERGEEVA S.A. ; KARKISHCHENKO V.N.** Pharmacokinetics. *Rostov-on-Don,* 2001, 383
- **MIROSHNICHENKO I.I.** Basics of pharmacokinetics. *M .: Publishing house "GEOTAR-MED,* 2002, 188
- **ROSIAK J. M. ; JANIK I. ; KADLUBOWSKI S. ; KOZICKI M. ; KUJAWA P. ; STASICA P. ; ULANSKI P.** Radiation formation of hydrogels for biomedical application. *Radiation synthesis and modification of polymers for biomedical applications Final results of a coordinated research project 1996-2000,* 2002, 5-48